(19) Europäisches Patentamt
European Patent Office
Office européen des brevets

(11) EP 1 248 610 B1

(12) **FASCICULE DE BREVET EUROPEEN**

(45) Date de publication et mention de la délivrance du brevet: **15.06.2005 Bulletin 2005/24**

(21) Numéro de dépôt: **01907633.0**

(22) Date de dépôt: **18.01.2001**

(51) Int Cl.[7]: **A61K 31/23**, A61P 35/00, A61K 7/06, A61K 7/48

(86) Numéro de dépôt international: **PCT/FR2001/000168**

(87) Numéro de publication internationale: **WO 2001/052837 (26.07.2001 Gazette 2001/30)**

(54) **UTILISATION D'AU MOINS UN ESTER GRAS POUR LA PREPARATION D'UNE COMPOSITION DESTINEE AU TRAITEMENT THERAPEUTIQUE ET/OU COSMETIQUE DE L'HYPERTROPHIE PROSTATIQUE, L'ADENOME PROSTATIQUE, L'ACNE, L'HYPERSEBORRHEE, L'ALOPECIE ET L'HIRSUTISME**

VERWENDUNG MINDESTENS EINES FETTSÄUREESTERS ZUR ZUBEREITUNG EINER PHARMAZEUTISCHEN ODER KOSMETISCHEN ZUSAMMENSETZUNG ZUR BEHANDLUNG VON PROSTATAHYPERTROPHIE, PROSTATA-ADENOM, AKNE, SEBORRHOE, HAARAUSFALL UND HIRSUTISMUS

USE OF AT LEAST A FATTY ESTER FOR PREPARING A COMPOSITION FOR THE THERAPEUTIC AND/OR COSMETIC TREATMENT OF PROSTATIC HYPERTROPHY, PROSTATIC ADENOCARCINOMA, ACNE, HYPERSEBORRHEA, ALOPECIA AND HIRSUTISM

(84) Etats contractants désignés:
**AT BE CH CY DE DK ES FI FR GB GR IE IT LI LU MC NL PT SE TR**

(30) Priorité: **18.01.2000 FR 0000577**

(43) Date de publication de la demande:
**16.10.2002 Bulletin 2002/42**

(73) Titulaire: **Laboratoires Expanscience**
**92419 Courbevoie Cedex (FR)**

(72) Inventeurs:
- **MSIKA, Philippe**
  **F-75018 Paris (FR)**
- **PICCIRILLI, Antoine**
  **F-78000 Versailles (FR)**
- **LEGRAND, Jacques**
  **F-61290 Neuilly sur Eure (FR)**

(74) Mandataire: **Warcoin, Jacques et al**
**Cabinet Régimbeau**
**20, rue de Chazelles**
**75847 Paris cedex 17 (FR)**

(56) Documents cités:

| | |
|---|---|
| **EP-A- 0 039 857** | **EP-A- 0 415 636** |
| **EP-A- 0 897 718** | **WO-A-94/01100** |
| **WO-A-98/13330** | **WO-A-98/52515** |
| **WO-A-99/22728** | **DE-A- 4 134 137** |

- **DATABASE WPI Section Ch, Week 199216 Derwent Publications Ltd., London, GB; Class D21, AN 1992-126448 XP002149392 & JP 04 066516 A (LION CORP), 2 mars 1992 (1992-03-02)**
- **DATABASE WPI Section Ch, Week 199937 Derwent Publications Ltd., London, GB; Class D21, AN 1992-187477 XP002149393 & JP 02 932642 B (LION CORP), 9 août 1999 (1999-08-09)**
- **LIANG T ET AL: "INHIBITION OF STEROID 5ALPHA-REDUCTASE BY SPECIFIC ALIPHATIC FATTY ACIDS" CLINICAL RESEARCH,US,THOROFARE, NJ, vol. 39, no. 3, 1991, page 720A XP000600261 ISSN: 0009-9279**
- **LIANG T ET AL: "INHIBITION OF STEROID 5ALPHA-REDUCTASE BY SPECIFIC ALIPHATIC UNSATURATED FATTY ACIDS" BIOCHEMICAL JOURNAL,GB,PORTLAND PRESS, LONDON, vol. 285, 1992, pages 557-562, XP000600136 ISSN: 0264-6021**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 248 610 B1

- **NIEDERPRUEM H -J ET AL: "Inhibition of steroid 5-alpha-reductase activity by aliphatic fatty acids. Candidates for chemoprevention of prostate cancer." ANNALS OF THE NEW YORK ACADEMY OF SCIENCES, vol. 768, 1995, pages 227-230, XP000949291 Conference;New York, New York, USA; September 22-24, 1994, the laboratory to the clinic: Implications of genetic, molecular, and preventive research. 1995 New York Academy of Sciences 2 East 63rd Street, New York, New York 10021, USA ISBN: 0-89766-947-9**

## Description

La présente invention se rapporte à l'utilisation d'au moins un ester gras pour la préparation d'une composition destinée à inhiber l'activité de la 5α-réductase, notamment pour le traitement de l'hypertrophie prostatique, de l'adénome prostatique, de l'acné, de l'hyperséborrhée, de l'alopécie, de l'hirsutisme. L'invention se rapporte également à des méthodes de traitement cosmétique, notamment de la peau grasse.

La 5α-réductase est une enzyme microsomiale NADPH dépendante qui existe sous forme de deux isoenzymes synthétisées à partir de deux gènes différents. L'isoenzyme de type 1 de la 5α-réductase est retrouvée essentiellement dans le foie et la peau, plus particulièrement dans les glandes sébacées de la peau non génitale et du cuir chevelu, et apparaît à la puberté. L'isoenzyme de type 2 est prédominante dans la prostate et au niveau de la peau des territoires sexuels différenciés : région génitale, barbe, et joue un rôle dans la différenciation sexuelle. La répartition des isoenzymes de type 1 et 2 de la 5α-réductase au niveau de la peau et des annexes cutanées chez l'homme peut être illustrée par le tableau suivant.

Tableau 1:

| **répartition des isoenzymes de type 1 et 2 de la 5α-réductase au niveau de la peau et des annexes cutanées chez l'homme** | | | |
|---|---|---|---|
| | | H5-a r1 | H5-a r2 |
| EPIDERME | Couche basale | ++ | + |
| | Couche spineuse | + | ++ |
| | Couche granuleuse | + | - |
| | Couche cornée | - | - |
| DERME | Fibroblastes | ++ | - |
| GLANDES SEBACEES | Cellules basales | ++ | + |
| | Cellules glandulaires | ++ | - |
| GLANDES SUDORALES ECCRINES | Canal excréteur | - | - |
| | Cellules sécrétrices | ++ | - |
| | Cellules myoépithéliales | ++ | + |
| FOLLICULE PILEUX | Papille dermique | + | + ? |
| | Cellules de la matrice | ++ | + |
| | Gaine épithéliale interne | ± | +++ |
| | Gaine épithéliale externe | ++ | - |
| | Muscle arrecteur | + | - |

Il existe un certain nombre de pathologies pour lesquelles une exagération congénitale ou acquise de l'activité de la 5α-réductase est responsable en totalité ou en majorité des troubles observés.

Par exemple, chez l'homme, cette enzyme 5α-réductase, principalement localisée dans les tissus génitaux et dans la peau, catalyse l'hydroxylation de la testostérone en 5α-réductase dihydrotestostérone (DHT). Or, comme la DHT est un androgène bien plus actif que la testostérone (environ 2 fois plus), les effets de cette dernière sont amplifiés dans les tissus où est produite la DHT. Une activité trop élevée de la 5α-réductase provoque ainsi des teneurs en androgène sous forme de DHT trop élevées dans la prostate, d'où une surstimulation de cette dernière se traduisant en une croissance indésirable pouvant mener à la pathologie de l'hypertrophie prostatique, voire à l'adénome prostatique, nécessitant le plus souvent une intervention chirurgicale.

D'autres pathologies, de type dennatologique, peuvent être observées chez l'homme ou la femme comme résultant d'une suractivité de la 5α-réductase à savoir, en particulier l'acné, l'hirsutisme ou encore l'alopécie.

Dans la peau, l'activité de la 5α-réductase est plus importante dans la glande sébacée que dans les autres structures. Par ailleurs, les glandes séborrhéiques montrent une activé 5α-réductase plus importante que celles des autres territoires cutanés. Par conséquent, le niveau de sécrétion sébacée physiologique semble étroitement lié à l'activité de cette enzyme.

Chez l'acnéïque, il existe une hyperactivité de la 5α-réductase. Plus qu'une augmentation des taux sériques

des androgènes, c'est une augmentation des précurseurs en DHT, facteur principal de la fonction sébacée, qui participent à l'acné. La peau grasse ou (séborrhée), outre son aspect disgracieux, constitue un terrain sur lequel peuvent survenir des complications. Elle atteint les zones où les glandes sébacées sont nombreuses et résulte principalement d'une surstimulation androgénique de la production sébacée par ces glandes spécifiques. L'hyperséborrhée participe à la survenue des lésions d'acné vulgaire.

Dans le cuir chevelu, on retrouve l'isoenzyme de type 1 de la 5α-réductase au niveau des glandes sébacées, ainsi qu'au niveau du follicule pileux L'isoenzyme de type 2 de la 5α-réductase est localisée majoritairement au niveau de la gaine épithéliale interne, ainsi qu'au niveau de la papille dermique du cheveu. Cependant cette dernière localisation reste à préciser.

L'alopécie androgénique, dont la physiopathogénie est très voisine de celle de l'acné, est la plus fréquente des alopécies et sans doute celle où la demande de thérapeutique est la plus forte. La 5α-réductase semble jouer un rôle primordial dans cette pathologie. En effet, les hommes atteints d'un déficit génétique en isoenzyme de type 2 de la 5α-réductase ne développent pas d'alopécie androgénétique.

Compte tenu de ce qui précède, la recherche s'est orientée vers la mise au point d'inhibiteurs de la 5α-réductase. Certains stéroïdes comme la progestérone ont été testés dans ce sens, mais sa métabolisation rapide la rend inefficace *in vivo.* Pour être actif, l'inhibiteur de 5α-réductase doit être suffisamment stable pour bloquer l'activité de l'enzyme *in vitro.* Le finastéride, inhibiteur compétitif stéroidien, rempli cette condition, mais il est plus actif sur l'isoenzyme de type 2 que sur l'isoenzyme de type 1 et ces deux isoenzymes n'ont que 50 % d'homologie sur la séquence de leurs acides aminés. C'est donc surtout dans l'hyperplasie bénigne de la prostate que le finastéride a déjà été testé.

Par ailleurs, on connaît également l'extrait de *Serenoa Repens,* comme référence en tant qu'inhibiteur de la 5α-réductase, l'extrait de *Serenoa Repens* présentant l'avantage, par rapport au finastéride, d'une origine naturelle en tant qu'extrait végétal permettant une meilleure comparaison pour des produits testés également d'origine naturelle. *Serenoa Repens,* également connu sous la dénomination *Sabal serrulatum*, est un petit palmier que l'on peut trouver aux Etats-Unis (Floride) en Afrique du Nord et en Espagne.

Des acides gras ont également été utilisés pour leurs propriété régulatrices de l'activité de la 5α-réductase. Ainsi, la demande de brevet DE 41 34137 décrit une lotion pour empêcher la chute des cheveux et comprenant du palmitate d'isopropyle. D'autres esters d'acides gras à dix atomes de carbone ont été utilisés pour lutter contre l'acné (EP-0 039 857). Les acides linoléniques, linoléiques et oléiques ont aussi été étudiés pour leurs propriétés inhibitrices de la 5α-réductase (WO-99/22728).

On a maintenant trouvé de manière tout à fait surprenante et inattendue que l'utilisation de certains esters gras, permet d'obtenir un effet remarquable d'inhibition de l'activité de la 5α-réductase procurant ainsi notamment une nouvelle réponse pour le traitement des pathologies et/ou désordres dermatologiques évoqués ci-dessus.

La présente invention se rapporte ainsi à l'utilisation d'au moins un ester gras choisi parmi les esters alkyliques de l'huile d'avocat, l'oléate de méthyle, le linoléate de méthyle, le stéarate de méthyle, l'oléate de butyle, l'oléate d'oléyle, le ricinoléate de méthyle, et les mélanges de ces derniers pour la préparation d'une composition destinée à inhiber l'activité de la 5α-réductase.

Ainsi, l'utilisation selon l'invention est caractérisée en ce que la composition est destinée au traitement de l hypertrophie prostatique de l'adénome prostatique, de l'acné, de l'hyperséborrhée, de l'alopécie, de l'hirsutisme.

La préparation des esters gras utilisables selon l'invention relève des méthodes connues de l'homme du métier. Les méthodes de préparation des esters non citées dans ce qui suit mais qui font partie des connaissances générales de l'homme du métier peuvent donc également être utilisées.

Bien entendu, on peut citer en premier lieu l'estérification d'un acide gras dont la chaîne grasse correspond à celle de l'ester gras désiré, avec l'alcool dont la partie hydrocarbonée correspond à celle du groupe alkoxy de l'ester gras désiré.

La formation des esters à partir des acides carboxyliques et des alcools peut se faire soit directement, soit en transformant l'acide en dérivés plus réactifs, soit en activant l'alcool pour une condensation avec le carboxylate.

L'estérification directe d'un acide carboxylique par un alcool est une réaction équilibrée, catalysée par les acides protoniques forts. La réaction est réalisée avec un large excès d'alcool dans un solvant qui forme un azéotrope éliminée par distillation. Les esters méthyliques peuvent être obtenus à partir de solutions de méthanol chlorhydrique à 5% ou sulfurique à 1-3%. On peut utiliser les acides de Lewis comme catalyseurs. On peut également citer le cas particulier de l'estérification directe entre un acide gras et le glycérol en présence de catalyseurs acides homogènes ou hétérogènes.

L'activation du carbonyle consiste à transformer l'hydroxyle en meilleur groupe partant. Les chlorures d'acides sont les dérivés les plus utilisés. On peut également citer l'utilisation des anhydrides d'acides.

L'activation de l'alcool consiste à rendre celui-ci plus réactif vis-à-vis d'une attaque nucléophile de l'ion carboxylate. Le diazométhane est une source très réactive de méthyl après déprotonation de l'acide.

Enfin, l'estérification des acides carboxyliques peut également être réalisée par voie enzymatique. Les lipases

catalysent l'estérification. Ces lipases proviennent de micro-organismes des genres *Aspergillus, Candida, Geotrichum, Mucor, Penicillium.*

Par ailleurs, les esters gras utilisables selon l'invention peuvent être préparés par transestérification qui est une réaction entre un ester et un alcool conduisant à un ester différent. Sous le terme de transestérification sont regroupés trois types de réaction :

- l'acoolyse qui est une réaction entre un ester et un alcool ;
- l'acidolyse qui est une réaction entre un ester et un acide carboxylique (ou un anhydride d'acide) ;
- l'interestérification qui est une réaction d'échange entre deux esters, des groupes non alkoxy.

La transestérification est avantageusement catalysée, par une catalyse homogène ou hétérogène.

De nombreux catalyseurs homogènes (acides ou basiques) sont décrits dans la littérature. Les catalyseurs acides peuvent être des acides minéraux forts tels que l'acide sulfurique, sulfonique, phosphorique ou hypochlorique mais également des acides organiques comme l'acide paratoluène sulfonique ou méthane sulfonique. Les catalyseurs basiques couramment employés en transestérification sont des bases classiques telles que des hydroxydes (soudes ou potasses) des carbonates ($K_2CO_3$, $Na_2CO_3$) ainsi que des alcoolates (Na $OCH_3$, NaOEt).

En catalyse hétérogène pour la transestérification, on peut utiliser les acides de Lewis mais ils sont en général peu actifs. Il a également été montré que l'alcoolate de tributylétain ($Bu_3SnOR$) pouvait catalyser la réaction de transestérification entre un alcool et un ester à 120°C pour un rapport molaire alcool/ester de 10. Les alcoolates de titane ($Ti(OR)_4$) sont utilisés depuis longtemps dans l'industrie comme catalyseurs actifs de transestérification. Cependant, il est nécessaire de les hydrolyser et de les filtrer en fin de réaction pour les éliminer du milieu réactionnel. En particulier, les titanates supportés se sont révélés actifs lors de la méthanolyse de l'huile de soja.

Parmi les autres catalyseurs pouvant être utilisés pour une catalyse hétérogène de la transestérification, on peut encore citer les guanidines, les lanthanides, les enzymes tels que les lipases comme *Pseudomanos sp* ou *Mercor Miehei,* les oxydes alcolino-terreux, l'oxyde de magnésium et enfin les catalyseurs supportés tels que des centres basiques (oxyde de calcium) supportés sur des oxydes variés (oxydes de magnésium, alumine, silice, etc.).

La catalyse hétérogène présente les avantages, par rapport à la catalyse homogène, de mettre en oeuvre des catalyseurs plus facilement séparables du milieu réactionnel et de susciter moins de problèmes de corrosion et de réactions secondaires.

Les produits de départ pour les réactions d'estérification décrites ci-dessus peuvent être d'origine synthétique ou naturelle, en particulier d'origine animale ou végétale.

On préfère les corps gras d'origine végétale ou animale comme produits de départ fournissant la chaîne grasse des esters gras utilisés selon l'invention.

On peut ainsi procéder par une estérification comme décrit ci-dessus d'un acide gras notamment d'origine végétale avec un alcool.

Parmi les acides gras utilisables pour ces réactions d'estérification, on citera en particulier les acides gras obtenus à partir des savons d'acide gras qui sont des sous-produits de la saponification d'une huile végétale d'avocat. Il s'agit en effet d'une valorisation très intéressante de ces sous-produits de la préparation des insaponifiables d'huile végétale.

La saponification de l'huile d'avocat est une étape essentielle du procédé d'obtention des insaponifiables. Cette étape, réalisée en présence de potasse aqueuse et d'éthanol, est une hydrolyse basique de l'huile (triglycérides) conduisant à la formation de savons de potassium et de glycérol :

$$\underset{\text{Triglycérides}}{(RCO_2)CH_2CH(O_2CR)CH_2(O_2CR)} + \underset{\text{Potasse}}{3\,KOH} \longrightarrow \underset{\text{Savons}}{3\,RCOO^-K^+} + \underset{\text{Glycérol}}{HOCH_2CH(OH)CH_2OH}$$

L'insaponifiable, en émulsion dans la phase hydro-alcoolique (phase "savonneuse"), est ensuite extrait par le dichloroéthane (DCE) selon un procédé d'extraction liquide-liquide. Après extraction, la phase hydro-alcoolique, débarassée de la fraction insaponifiable, est un mélange constitué essentiellement de savons, d'éthanol, d'eau, de glycérol, de DCE et de fraction I. La fraction I est une des composantes de l'insaponifiable d'avocat. Elle est constituée de substrats présentant une chaîne alkyle grasse (radical R) et des fonctions hydroxyles :

$$RCH_2\text{-}CH(OH)CH_2CH(OH)CH_2OH$$

Ces composés hydroxylés sont partiellement solubles dans la phase hydro-alcoolique.

Après l'étape d'extraction liquide-liquide, la phase "savonneuse" est acidifiée par l'acide sulfurique. Les savons sont alors transformés en acides gras (réaction 1 ci-dessous). Le mélange obtenu est ensuite distillé afin d'éliminer l'éthanol et les traces de DCE. Les acides gras et l'eau sont enfin séparés par décantation.

$$2\ RCOO^-K^+ + H_2SO_4 \rightarrow 2\ RCOOH + K_2SO_4 \qquad (1)$$

Ces acides gras bruts d'avocat sont enfin purifiés, par exemple sur une colonne de silice (éluant hexane puis hexane-éther diéthylique 95:5) ou par distillation moléculaire et peuvent ainsi constituer la matière première mise en oeuvre lors de la synthèse des esters gras d'avocat, notamment des esters méthyliques d'avocat.

Ainsi, selon un mode de réalisation particulier de réalisation, l'ester gras utilisé selon l'invention est obtenu par estérification d'au moins un acide gras d'une huile végétale d'avocat, étant entendu que l'expression "acide gras d'huile végétale" englobe selon l'invention les acides gras présents à l'origine dans ladite huile végétale et les acides gras pouvant être obtenus par traitement de la phase savonneuse après saponification de ladite huile végétale, comme décrit ci-dessus.

Les triglycérides présents dans les corps gras d'origine végétale (notamment les huiles végétales) peuvent également représenter une source non négligeable d'esters gras utilisables selon l'invention, par le biais d'une transestérification en utilisant des moyens connus de l'homme du métier comme décrit ci-dessus.

Ainsi, selon un autre mode particulier de réalisation, l'ester gras utilisé selon l'invention est obtenu par transestérification des triglycérides d'au moins une huile végétale d'avocat.

Par "esters alkyliques de l'huile d'avocat", on entend selon l'invention un mélange d'esters gras obtenu par estérification d'acides gras d'huile d'avocat, les acides gras ayant été obtenus par traitement de la phase savonneuse après saponification de l'huile d'avocat et soumis ensuite à une estérification avec au moins un alcool ou un mélange d'alcools, dont le groupe alkyle détermine la partie "alkylique" de ces esters alkyliques. Par exemple, l'utilsation de butanol permet ainsi d'obtenir des esters "butyliques". De préférence, on utilise un alcool ayant de 1 à 6 atomes de carbones, dont le groupe alkyle est linéaire ou ramifié, comme par exemple l'isopropanol. On comprend bien entendu que cette définition englobe l'utilisation pour une telle estérification d'un mélange d'alcools comme déjà mentionné ci-dessus, par exemple d'un mélange de butanol et de méthanol. De préférence encore, on réalise une telle estérification avec un mélange catalytique notamment un mélange $BF_3$/éther éthylique.

Plus particulièrement, l'ester gras est constitué des esters méthyliques de l'huile d'avocat. Par "esters méthyliques de l'huile d'avocat", on entend selon l'invention un mélange d'esters gras obtenu par estérification d'acides gras d'huile d'avocat, les acides gras ayant été obtenus par traitement de la phase savonneuse après saponification de l'huile d'avocat et soumis ensuite à une estérification avec du méthanol, de préférence avec un mélange catalytique notamment un mélange $BF_3$/éther éthylique.

Plus particulièrement encore, l'ester gras est constitué des esters butyliques de l'huile d'avocat. Par "esters butyliques de l'huile d'avocat", on entend donc selon l'invention un mélange d'esters gras obtenu par estérification d'acides gras d'huile d'avocat, les acides gras ayant été obtenus par traitement de la phase savonneuse après saponification de l'huile d'avocat et soumis ensuite à une estérification avec du butanol, de préférence avec un mélange catalytique notamment un mélange $BF_3$/éther éthylique. Les esters butyliques d'huiles d'avocat se caractérisent comme suit :

| **Esters butyliques d'huile d'avocat** | |
|---|---|
| Teneurs en esters butyliques | 95% min. |
| Indice d'acide | < 5 |
| Insaponifiable résiduel | < 0.5% wt |
| | |
| Répartition en acides gras : | |
| Acide palmitique C16 | 12 à 25% |
| Acide palmitoléique C 16' | 3 à 10% |
| Acide stéarique C18 | Traces |
| Acide oléique C18' | 45 à 75% |
| Acide linoléique | 6 à 18% |
| Acide linolénique C18' | < 5% |

Selon l'invention, l'ester gras tel que décrit ci-dessus est utilisé selon une proportion comprise entre 0,001 et

100 % en poids (utilisation sous forme pure par exemple en tant qu'émollient), de préférence entre 0,01 et 70 % en poids, et plus particulièrement encore entre environ 0,1 et 10 % en poids, par rapport au poids total de la composition.

La composition préparée par l'utilisation selon l'invention peut en outre comprendre un excipient pharmaceutiquement, dermatologiquement ou cosmétiquement acceptable. On peut utiliser tout excipient adapté pour les formes galéniques connues de l'homme de métier, en vue d'une administration par voie topique, orale, entérale ou parentérale, notamment rectale.

En particulier, cet excipient peut être adapté pour l'obtention d'une composition sous forme d'une solution huileuse, d'une émulsion eâu-dans-huile, une émulsion huile-dans-eau, une microémulsion, un gel huileux, un gel anhydre, une crème, une dispersion de vésicules, de microcapsules ou de microparticules, ou encore de gellules ou de capsules molles de gélatine ou végétales.

De préférence, on utilise un excipient adapté pour une administration par voie topique externe ou par voie rectale.

L'effet avantageux d'inhibition de l'activité de la 5$\alpha$-réductase fourni par l'utilisation selon l'invention permet de destiner la composition ainsi préparée à des traitements thérapeutiques, notamment dermatologiques, et cosmétiques.

L'utilisation d'un excipient adapté pour une administration par voie rectale comme décrit ci-dessus peut être particulièrement envisagée pour ces traitements de l'hypertrophie et/ou de l'adénome prostatique.

La présente invention a encore pour objet une méthode de traitement cosmétique de la peau grasse, caractérisée en ce qu'on applique sur la peau une composition cosmétique contenant au moins un ester gras tel que décrit ci-dessus. L'invention a par ailleurs pour objet une méthode de traitement cosmétique de la chute des cheveux, caractérisée en ce qu'on applique sur le cuir chevelu une composition cosmétique contenant au moins un ester gras tel que décrit ci-dessus.

Enfin, l'invention a également pour objet une méthode de traitement cosmétique de l'excès de pilosité, caractérisée en ce qu'on applique sur les zones de la peau présentant des excès de pilosité une composition cosmétique contenant au moins un ester gras tel que décrit ci-dessus.

En effet, à l'opposé des traitements médicaux hormonaux, ces deux dernières méthodes de traitement cosmétique permettent d'améliorer l'apparence en réduisant de manière visible les phénomènes disgracieux de chute de cheveux liés à l'alopécie et les phénomènes d'excès de pilosités liés à l'hirsutisme.

Selon un mode de mise en oeuvre préféré de ces méthodes de traitements cosmétiques, l'ester gras est présent dans la composition selon une proportion comprise entre 0,001 et 100 % en poids (utilisation sous forme pure, sans excipient, par exemple en tant qu'émollient), de préférence entre 0,01 et 70 % en poids, et plus particulièrement encore entre environ 0,1 et 10 % en poids, par rapport au poids total de la composition.

Avantageusement, la composition cosmétique appliquée selon la méthode cosmétique de l'invention contient en outre au moins un excipient cosmétiquement acceptable tel que décrit ci-dessus.

Les exemples suivants sont destinés à illustrer la présente invention.

A moins qu'il n'en soit précisé autrement, les pourcentages indiqués dans les exemples suivants sont des pourcentages en poids.

**Exemple 1 : préparation d'esters méthyliques d'huile d'avocat**

On prépare des esters méthyliques d'huile d'avocat selon le mode opératoire suivant.

**1. Obtention des acides gras d'avocat purifiés**

La saponification de l'huile d'avocat est une étape essentielle du procédé d'obtention des insaponifiables. Cette étape, réalisée en présence de potasse aqueuse et d'éthanol, est une hydrolyse basique de l'huile (triglycérides) conduisant à la formation de savons de potassium et de glycérol :

$$(RCO_2)CH_2CH(O_2CR)CH_2(O_2CR \quad + \quad 3\ KOH \quad \longrightarrow \quad 3\ RCOO^-K^+ \quad + \quad HOCH_2CH(OH)CH_2OH$$

| Triglycérides | Potasse | Savons | Glycérol |
|---|---|---|---|

L'insaponifiable, en émulsion dans la phase hydro-alcoolique (phase "savonneuse"), est ensuite extrait par le dichloroéthane (DCE) selon un procédé d'extraction liquide-liquide. Après extraction, la phase hydro-alcoolique, débarrassée de la fraction insaponifiable, est un mélange constitué essentiellement de savons, d'éthanol, d'eau, de glycérol, de DCE et de fraction I. La fraction I est une des composantes de l'insaponifiable d'avocat. Elle est constituée

de substrats présentant une chaîne alkyle grasse (radical R) et des fonctions hydroxyles :

$$RCH_2\text{-}CH(OH)CH_2CH(OH)CH_2OH$$

Ces composés hydroxylés sont partiellement solubles dans la phase hydro-alcoolique.

Après l'étape d'extraction liquide-liquide, la phase "savonneuse" est acidifiée par l'acide sulfurique. Les savons sont alors transformés en acides gras (réaction 1). Le mélange obtenu est ensuite distillé afin d'éliminer l'éthanol et les traces de DCE. Les acides gras et l'eau sont enfin séparés par décantation.

$$2\ RCOO^-K^+ + H_2SO_4 \rightarrow 2\ RCOOH + K_2SO_4 \qquad (1)$$

Ces acides gras bruts d'avocat sont enfin purifiés sur une colonne de silice (éluant hexane puis hexane-éther diéthylique 95:5) et constituent de la sorte la matière première mise en oeuvre lors de la synthèse des esters méthyliques d'avocat.

**2. Préparation des esters méthyliques d'huile d'avocat**

Les esters méthyliques d'avocat sont obtenus selon le mode opératoire suivant :

Dans un ballon tricol, équipé d'un réfrigérant et d'une agitation mécanique, on mélange 250 ml de méthanol, 500 g d'acides gras d'avocat et 12,5 ml d'un mélange BF3/éther éthylique. Le mélange réactionnel est alors porté à reflux pendant 1 heure.

Les esters méthyliques ainsi obtenus sont séchés sous vide à l'évaporateur rotatif puis enfin purifiés par distillation moléculaire, à 120°C sous un vide de 4 µm de mercure et avec un taux de distillation de 90 %.

On peut par exemple également obtenir des esters butyliques respectivement d'huile d'avocat selon la même voie de synthèse excepté que l'on remplace le méthanol par du butanol (voir l'exemple 6 de composition ci-après).

**3. Analyse des produits obtenus**

**3.1 Composition des acides gras d'avocat purifiés**

Tableau 2

| | |
|---|---|
| Teneur en Acides Gras | 96,2 % |
| Insaponifiable résiduel | 3,8 % |
| | |
| Répartition en Acides Gras : | |
| Acide myristique C14:0 | 0,08 % |
| Acide palmitique C16:0 | 22,7 % |
| Acide palmitoléique C16:1 | 9,9 % |
| Acide stéarique C18:0 | 0,6 % |
| Acide oléique C18:1 | 50,3 % |
| Acide linoléique C18 : 2 | 11,8 % |
| Acide linolénique C18 :3 | 0,9 % |

**3.2 Composition des esters méthyliques d'avocat**

Tableau 3

| | |
|---|---|
| Esters Méthyliques d'Acides Gras | 86,0 |

Tableau 3 (suite)

| | |
|---|---|
| Insaponifiable résiduel | 2,1 |
| Composants non déterminés | 11,9 |
| | |
| Répartition en acides gras : | |
| Acide palmitique C16:0 | 25,1 % |
| Acide palmitoléique C16:1 | 8,4 % |
| Acide stéarique C18:0 | 0,6 % |
| Acide oléique C18:1 | 55,8 % |
| Acide linoléique C18:2 | 9,1 % |
| Acide linolénique C18:3 | 0,4 % |

**Exemple 2 : Evaluation de l'activité inhibitrice sur l'activité de la 5α-réductase par mesure du taux de 5-dihydrotestostérone formée à partir de la testostérone par les cellules DU145.**

**1. Matériel et méthodes**

**1.1 Matériel**

Les cellules prostatiques DU145 sont issues d'une lignée tumorale obtenue à partir d'un carcinome de la prostate (N° ATCC HTB 81). Le milieu MEM (réf.0410265), la glutamine et la gentamycine viennent de chez Gibco. Le sérum de veau foetal (SVF) vient de chez DAP et est utilisé décomplémenté (45 mm à 56°C). les plastiques servant à la culture (boîtes et plaques) viennent de chez Costar. La testostérone vient de chez Sigma.

**1.2 Méthode**

**1.2.1 Préparation des gammes de produits**

Une solution mère en éthanol à 10 mg/ml est préparée à partir de chacun des produits testés.
La gamme de concentration utilisée pour les expériences est la suivante : 0, 5, 10, 50, 100 et 500 microgrammes/ml. (Dilution effectuée dans le milieu de culture).
Le volume d'extrait ajouté par puits étant de 20 microlitres/puits, les solutions à préparer sont concentrées 50X.

<u>Préparation de la Testostérone</u>

Une solution mère de testostérone à 10 mM est préparée dans l'éthanol. Au moment de son utilisation, cette solution est diluée au 1:1000 dans le milieu de culture et 10 micro litres_sont ajoutés par puits.

**1.2.2. Expérience d'inhibition de la 5α- réductase des DU 145**

Les cellules prostatiques DU145 sont cultivées à 37°C, 5% $CO_2$ dans un milieu MEM contenant de la glutamine (2mM), de la gentamycine (50 microgrammes/ml) et 10% de SVF. Leur taux de sous-culture est de 1:10.
Avant de lancer l'expérimentation, les cellules sont mises en culture dans des plaques 6 puits à raison de $2.10^5$ DU145 par puits/1 ml de milieu ne contenant que 1% de SVF. Les cellules sont maintenues 3 jours à 37°C, 5% $CO_2$. Le jour de l'expérience, le milieu de culture contenu dans les puits est éliminé et remplacé par du milieu neuf contenant 1% de SVF. La testostérone (0,1 micromolaire final) ainsi que les extraits aux différentes concentrations sont ajoutés au milieu à raison de 10 et 20 microlitres/puits respectivement. (Les puits "contrôles" correspondent à des cellules incubées en présence de testostérone et d'un équivalent Ethanol. Ceci permet de soustraire l'effet du solvant sur les cultures et de déterminer le pourcentage de DHT formée en absence d'inhibiteur). Les cellules sont alors incubées à 37°C, 5% $CO_2$. Au bout de 3 heures, les surnageants de culture sont collectés et congelés à -80°C jusqu'au dosage.

**Mesure du taux de DHT formée**

Principe: extraction des produits lipophiles par l'éther, concentration des échantillons en DHT par chromatographie d'affinité et dosage radio-immunologique

Préparation des échantillons

- Après avoir agité au vortex les prélèvements, introduire les échantillons dans des flacons "SEPEX"
- Ajouter dans chaque tube 0,1 ml de la solution radioactive"3H-Rdt" (pour évaluation du rendement d'extraction). Boucher les flacons, les agiter un par un au vortex.
- Laisser reposer 30 min à température ambiante. Puis agiter de nouveau chaque flacon au vortex.
- Ajouter dans chaque flacon : 5 ml d'éther éthylique.
- Boucher les flacons et les agiter manuellement de façon énergique. Laisser décanter quelques minutes.
- Congeler les phases aqueuses à -30°C, pendant au moins 1 heure.
- Recueillir la phase éthérée dans un tube à essai en boro-silicate de 5 ml correspondant.
- Evaporer totalement la phase éthérée à l'aide du système évaporateur + bain-marie à 37°C.

Séparation de la DHT

- Préparation des colonnes : Préparer les colonnes dans des pipettes de culture en verre de 5 ml avec 10 cm de chromatolithe A.
- Rinçage des colonnes : 3 ml d'iso-octane pur combitips (3 fois), en laissant couler par simple gravité.
- Elution des extraits éthérés secs
    - Chaque extrait sec est repris par 1 ml d'iso-octane pur, vortexer vigoureusement. Attendre 15 min à température ambiante. Réagiter au vortex.
    - Lorsque les 3 ml d'iso-octane (lavage des colonnes) sont élues, transvaser les extraits éthérés secs repris par l'iso-octane sur la colonne. Laisser éluer.
    - Rincer chaque tube "extrait sec" avec 1 ml d'iso-octane pur combitips, vortexer vigoureusement. Attendre 15 min à température ambiante. Réagiter au vortex et transvaser dans la colonne comme précédemment.
    - Laver par 4 ml d'iso-octane pur.
- Recueil de la DHT
    - Préparer le solvant d'élution (mélange à 6% iso-octane/acétate d'éthyl: 94/6 (v-v))
    - Eluer par 6 ml (pipette) de ce mélange.
    - Recueillir l'éluat DHT dans les tubes à essais en boro-silicate de 5 ml identifiés.
- Traitement de l'éluat DHT : Evaporer le solvant de l'éluat à l'aide du système évaporateur-bain-marie (37°C)

Dosage RIA

- Protocole de distribution : Rependre les échantillons par 0,5 ml de tampon RC, le Blanc par 1 ml de tampon Rcet, les Contrôles par 0,5 ml de tampon RC. Placer à l'étuve à 37°C 15 min Agiter de nouveau les tubes au sortir de l'étuve (1 min).

    Dans des tubes à hémolyse en verre identifiés de 5 ml, mettre dans l'ordre:

    * **Tampon :** Activité Totale (AT): 0,7 ml de tampon RC, Activité Non Spécifique (N): 0,2 ml de tampon RC, Gamme : seul le point 0 de la gamme (noté B0) comporte 0,1 ml de tampon RC,
    * **Solution étalon** (1000 à 7,8 pg/tube) : 0,1 ml de la solution étalon respective.
    * 0,1 ml d'extrait sec repris dans le tampon
        - Puis, distribuer **l'anti-sérum :** 0,1 ml dans tous les tubes sauf AT et N.
        - Puis, distribuer **la solution de dosage "3HD" :** 0,1 ml dans tous les tubes.
        - Vortexer et recouvrir d'un parafilm.
        - Incubation à 4°C, pendant 1h30 minimum (24 h. maximum).

- Préparation du charbon-dextran : Mettre la suspension de charbon-dextran dans un bêcher, puis, dans un bain d'eau glacé à 4°C, pendant au moins 1h30.
- Rendement de purification en DHT
  - Dans 6 petites fioles à scintillation (3 par série) déposer : 0,4 ml de tampon RC + 0,1 ml de la solution "3H-Rdt" (flacon du premier jour au réfrigérateur). Blancs : mettre 0,5 ml d'extrait sec reconstitué pour le blanc. Echantillons et contrôles : mettre 0,25 ml de tampon RC + 0,25 ml d'extrait.
  - Ajouter 5 ml de liquide à scintillation dans toutes les fioles.

Séparation de la DHT libre de celle liée à l'anticorps

- Mettre la suspension de charbon-dextran sous agitation magnétique, dans une bassine d'eau glacée.
- Ajouter 0,5 ml de charbon-dextran dans tous les tubes sauf AT en 2 min maximum.
- Vortexer, remettre les tubes dans l'eau glacée. Attendre 10 min exactement. Centrifuger à 4°C, 3400 rpm, pendant 11 min
- Pipeter 0,5 ml de chaque surnageant (y compris AT) dans une petite fiole de comptage
- Ajouter 5 ml de liquide scintillant. Agiter, laisser s'équilibrer 30 min à température ambiante.
- Mettre à compter 2 min avec le compteur β (Beclanan, LS 6000 SE).

**2. Résultats**

**2.1 Evaluation de la conversion de la testostérone en 5-dihydrotestostérone par les cellules DU 145 - Détermination des IC 50**

Tableau 5

| Produit testé | IC 50 (μg/ml) |
|---|---|
| Acides gras d'huile d'avocat (1) | 510 |
| Esters méthyliques d'huile d'avocat (1) | 13 |
| Oléate de méthyle (2) | 386 |
| Serenoa repens | 60 |

(1) Obtenus comme décrit à l'exemple 1
(2) Produit commercial (Sigma, pureté 99 %)

**3. Conclusions**

De façon générale, les esters méthyliques d'acides gras présentent une activité inhibitrice de la 5α-réductase.

Parmi eux, les esters méthyliques d'huile d'avocat constituent le produit le plus actif. Ils sont 5 à 6 fois plus actifs que l'extrait de Serenoa repens, inhibiteur reconnu de la 5α-réductase.

Les esters méthyliques d'avocat présentent une activité inhibitrice de la 5-alpha-réductase contrairement à leurs précurseurs, les acides gras d'avocat, qui présentent une activité nettement plus faible sur cette enzyme.

Enfin, l'oléate de méthyle, constituant majoritaire des esters méthyliques d'avocat (50%), testé seul, s'avère nettement moins actif que les esters méthyliques d'avocat.

**Exemple 3 : Evaluation in vitro de l'activité de la 5-α réductase sur la conversion de la testostérone en 5α-dihydrotestostérone dans des cultures de fibroblastes dermiques humains normaux.**

**Abréviations utilisées dans les exemples suivants :**

$^3H$ : tritium

| | |
|---|---|
| CCM : | chromatographie en couche mince |
| Ci | : Curie |
| DMSO : | diméthyl sulfoxyde |
| M199 | : appellation donnée à un milieu de culture standard |
| MCF | : milieu de culture des fibroblastes |
| MEM : | *medium* appellation donnée au milieu de culture, *Minimum Essential* |
| MIF | : milieu d'incubation des fibroblastes |
| Rf | : facteur de rétention relatif |
| SVF | : sérum de veau foetal |
| 5α-DHT : | 5α-dihydrotestostérone |

On se propose d'évaluer l'effet des produits tels que l'oléate de méthyle (produit commercial Sigma, pureté 99 %), des esters méthyliques d'avocat et d'un extrait de Serenoa Repens choisi comme référence, sur l'activité de la 5α-réductase. Un modèle *in vitro* de cultures de fibroblastes dermiques humains normaux a été retenu.

**1. Matériels et méthodes**

**1.1 Produits à l'essai, produit de référence, et réactifs**

Les produits à l'essai ont été fournis par EXPANSCIENCE et ont été conservés à +4°C jusqu'au moment de leur utilisation.
La testostérone radioactive (marquée au tritium en position 1, 2, 6 et 7, activité spécifique 79 Ci/mmol) était fournie par AMERSHAM, la testostérone non-radiomarquée était fournie par SIGMA.
Les réactifs de qualité analytique, provenaient de chez SIGMA, MERCK, BDH, ALDRICH ou CARLO ERBA sauf indication contraire.

**1.2 Système d'essai**

Le milieu de culture des fibroblastes (MCF) était constitué par du MEM/M199 (3:1, v/v) additionné de pénicilline (50 UI/ml), de streptomycine (50 µg/ml), de bicarbonate de sodium (0,2 %, p/v) et de SVF (10 %, v/v).
Le système d'essai était constitué de fibroblastes dermiques humains normaux cultivés en monocouche. Les fibroblastes ont été isolés à partir d'un résidu de plastie abdominale réalisée chez une femme de 51 ans (sujet BIOPREDIC n°I0013). Les cellules ont été utilisées au cinquième passage, elles ont été cultivées jusqu'à confluence des monocouches dans le milieu MCF à 37°C dans une atmosphère humide contenant 5 % de $CO_2$.

**1.3 Préparation des produits et incubation avec le système d'essai**

Le milieu d'incubation des fibroblastes (MIF) était constitué de MCF additionné de testostérone tritiée (1,6 x $10^{-7}$ M, soit 6,32 µCi/ml) et de testostérone non radiomarquée (3,84 x $10^{-6}$ M).
Les produits à l'essai et le finastéride ont été repris dans du DMSO avant d'être dilué dans le milieu d'incubation. La concentration finale en DMSO a été maintenue constante et égale à 1% (v/v) dans chaque dilution de produits à l'essai et de produit de référence.

Echelle de temps :

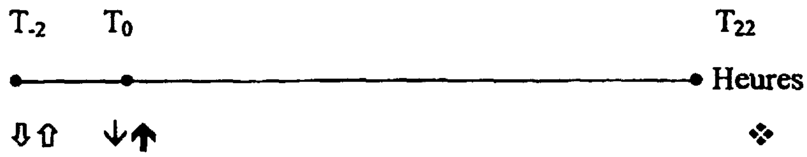


⇩ : élimination du milieu MCF

⇧ : pré-incubation des produits à l'essai et du produit de référence préparés dans le milieu MCF

↓ : élimination des milieux MCF contenant les produits à l'essai ou le produit de référence

🡅 : incubation des produits à l'essai et du produit de référence préparés dans le milieu MIF

❖ : détermination de l'activité de la 5α -réductase

Les cultures de fibroblastes ont été pré-incubées en présence des produits à l'essai ou du produit de référence pendant 2 heures avant l'ajout du substrat, la testostérone. Pour cette étape, les produits à l'essai et le produit de référence ont été préparés dans le milieu MCF.

Après la pré-incubation, les cultures de fibroblastes ont été incubées en présence des produits à l'essai ou du produit de référence préparés dans le milieu MIF pendant 22 heures à 37°C dans une atmosphère humide contenant 5 % de $CO_2$. Des cultures témoins ont été incubées dans le milieu MIF en absence de produits à l'essai et de produit de référence. Des cultures «témoin DMSO» ont été incubées dans le milieu MIF contenant 1% (v/v) de DMSO.

Chaque condition expérimentale a été testée en triplicate.

**1.4 Evaluation des effets**

Après la période d'incubation, les cellules ont été soumises à l'action des ultrasons dans le milieu MIF. Les lysats cellulaires ainsi obtenus ont été extraits par du dichlorométhane. Après évaporation, les résidus secs ont été repris dans du méthanol et ont été déposés sur des plaques de silice $60F_{254}$ (MERCK, référence 5554).

Des standards non radiomarqués, la testostérone, la 5α-dihydrotestostérone et l'androstènedione, ont été déposés sur chacune des plaques.

Le solvant de migration était un mélange de dichlorométhane et d'éther (7:3, v/v) A la fin de la migration, les plaques de silices ont été lues à l'aide d'un scanner de radioactivité BERTHOLD.

Les standards non radiomarqués ont été mis en évidence par pulvérisation d'acide sulfurique à 5 % (v/v) sur les plaques de chromatographie chauffées ensuite à 100°C pendant 10 minutes.

La comparaison des Rf (facteur de rétention relatif) déterminés pour les standards avec ceux obtenus pour les différents métabolites radioactifs a permis l'identification de ces derniers.

La métabolisation de la testostérone en 5α-dihydrotestostérone dans les différentes conditions expérimentales a été calculée : les résultats (aires des pics de 5α-dihydrotestostérone comptés par le scanner BERTHOLD) ont été exprimés en pmoles de 5α-dihydrotestostérone formées par puits de culture. Ils ont aussi été exprimés en pourcentage de l'activité 5α-réductase présente dans le groupe 'témoin DMSO'.

**1.5 Traitement des données**

Les groupes de données (groupe témoin et groupes traités) ont été traités par une analyse de la variance à un facteur (ANOVA 1, $p<0,05$), suivie par un test de DUNNETT ($p<0,05$). L'effet des produits à l'essai et du produit de référence a été comparé au groupe 'témoin DMSO'. Les effets des produits à l'essai ont été comparés entre eux par une analyse de la variance à deux facteurs (ANOVA 2, $p<0,05$, facteur 1 = concentration et facteur 2 = traitement).

## 2. Résultats et discussion

Dans les cultures témoins, la vitesse de métabolisation de la testostérone était de 9,71 +/- 0,77 pmoles de 5α-DHT formées en 22 heures par puits de culture. Cette vitesse était conforme aux résultats déjà obtenus au laboratoire.
Les esters méthyliques d'avocat, testés à 10 et100 μg/ml, inhibaient respectivement de 29 et 55 % l'activité de la 5α-réductase (tableau 6).
L'oléate de méthyle purifié, testé à 1, 10 et 100 μg/ml, inhibait respectivement de 24, 38 et 41% l'activité de la 5α-réductase (tableau 7).
L'extrait de *Serenoa Repens,* produit de référence, testé à 10 et 100 μg/ml, inhibait respectivement de 15 et 35% l'activité de la 5α-réductase. A 1 μg/ml, il n'avait pas d'effet (tableau 7).
En conclusion, les produits testés inhibaient l'activité de la 5α- réductase.
En fonction de l'activité des produits à l'essai, testés à 10 et 100 μg/ml, les esters méthyliques d'avocat et l'oléate de méthyle purifié présentaient une activité d'inhibition de la 5α-réductase significativement supérieure à celle de l'extrait de *Serenoa Repens,* choisi comme référence.
Ce test confirme donc l'activité inhibitrice des esters méthyliques d'acides gras, et en particulier ceux de l'huile d'avocat.

## 3. Tableaux

### 3.1 - Effet des esters méthyliques d'avocat sur l'activité de la 5α-réductase dans des cultures de fibroblastes dermiques humains normaux après 22 heures d'incubation

Tableau 6

| Produit | DMSO 1% (v/v) | Concentration (μg/ml) | |
|---|---|---|---|
| | | 10 | 100 |
| Esters méthyliques d'avocat | 8,00 | 5,80 | 4,28 |
| | 8,92 | 6,36 | 2,72 |
| | 8,68 | 6,08 | 4,48 |
| | **8,53+/-0,48** | **6,08*+/-0,28** | **3,83*+/-0,96** |
| | *100* | *71* | *45* |
| Les résultats sont exprimés en pmoles de 5α-DHT formées/puits de culture.<br>En gras : moyenne et écart type<br>En italique : pourcentage du groupe DMSO | | | |

* : moyenne significativement différente du groupe DMSO (p<0,05)

### 3.2 - Effet de l'oléate de méthyle et de l'extrait de Serenoa Repens sur l'activité de la 5α-réductase dans des cultures de fibroblastes dermiques humains normaux après 22 heures d'incubation.

Tableau 7

| Produit | DMSO 1% (v/v) | Concentration (μg/ml) | | |
|---|---|---|---|---|
| | | 1 | 10 | 100 |
| Oléate de méthyle | 8.00 | 7.12 | 3.00 | 5.44 |
| | 8,92 | 6,24 | 7,04 | 5,12 |
| | 8,68 | 6,08 | 5,92 | 4.60 |
| | **8,53+/-0,48** | **6,48*+/-0,56** | **5,32*+/-2,09** | **5,05*+/-0,42** |
| | *100* | *76* | *62* | *59* |
| Extrait de Serenoa Repens | 8.00 | 7.72 | 6.84 | 5.48 |
| | 8,92 | 9,20 | 7,48 | 5,68 |
| | 8,68 | 8,08 | 7,52 | 5,44 |
| | **8,53+/-0,48** | **8,33+/-0,77** | **7,28*+/-0,38** | **5,53*+/-0,13** |
| | *100* | *98* | *85* | *65* |
| Les résultats sont exprimés en pmoles de 5α-DHT formées/puits de culture.<br>En gras : moyenne et écart type | | | | |

* : moyenne significativement différente du groupe DMSO (p<0,05)

**Exemple 4 : Evaluation in vitro des esters d'acides gras sur l'activité de la 5α-réductase pour la conversion de la testostérone en 5α-dihydrotestostérone dans des cultures de fibroblastes dermiques humains normaux.**

**1. Matériels et méthodes**

On utilise les même matériels et méthodes qu'à l'exemple 3 précédent.

**2 - Résultats et discussion**

On s'est proposé d'évaluer l'effet des esters d'acides gras sur l'activité de la 5α-réductase. Un modèle *in vitro* de cultures de fibroblastes dermiques humains normaux a été retenu. Les produits testés ont été choisis dans le groupe des esters d'acides gras, variant par la longueur et la fonctionnalité de la chaîne grasse, et la nature du groupe alkoxy. Ces esters sont les suivants : l'oléate de méthyle, l'oléate de butyle, l'oléate d'oléyle, et le ricinoléate de méthyle.

L'oléate de méthyle, testé à 1 ; 10 et 100 μg/ml, inhibait significativement (p<0,05) de 29% ; 42% et 26% respectivement l'activité de la 5α-réductase.

L'oléate d'oléyle, testé à 1 ; 10 et 100 μg/ml, inhibait significativement (p<0,05) de 28% ; 45% et 35% respectivement l'activité de la 5α-réductase. A 10 et 100 μg/ml, les fibroblastes présentaient une souffrance cellulaire constatée par l'examen morphologique des cellules.

L'oléate de butyle, testé à 1 ; 10 et 100 μg/ml, inhibait significativement (p<0,05) de 21% ; 45% et 49% respectivement l'activité de la 5α-réductase.

En conclusion, dans les conditions expérimentales retenues, en fonction de l'activité des produits à l'essai, testés à 1 ; 10 et 100 μg/ml, il a été possible de mettre en évidence, pour les oléates de méthyle, de butyle, d'oléyle et le ricinoléate de méthyle, une activité d'inhibition de la 5α-réductase significative.

**3 - Tableaux de résultats détaillés : Effet des esters d'acides gras l'activité de la 5α-réductase dans des cultures de fibroblastes dermiques humains normaux, après 24 heures d'incubation**

Tableau 8

| Produit | DMSO 0,1% (v/v) | Concentration (µg/ml) | | |
|---|---|---|---|---|
| | | 1 | 10 | 100 |
| Oléate de méthyle | 10,56 | 8,24 | 6,28 | 7,52 |
| | 12,00 | 8,28 | 6,44 | 7,00 |
| | 11,64 | 7,84 | 7,12 | 7,20 |
| | **11,40+/-0,75** | **8,12*+/-0,24** | **6,61*+/-0,45** | **7,24*+/-0,26** |
| | *100* | *71* | *58* | *64* |
| | | | | |
| Oléate d'oleyle | 10,56 | 9,00 | 6,12 | 6,28 |
| | 12,00 | 7,32 | 6,44 | 8,60 |
| | 11,64 | 8,20 | 6,20 | 7,48 |
| | **11,40+/-0,75** | **8,17*+/-0,84** | **6,25*+/-0,17** | **7,45*+/-1,16** |
| | *100* | *72* | *55* | *65* |
| Oléate de butyle | 10,56 | 10,64 | 6,24 | 5,20 |
| | 12,00 | 8,28 | 5,04 | 6,72 |
| | 11,64 | 8,08 | 7,36 | 5,40 |
| | **11,40+/-0,75** | **9,00*+/-1,42** | **6,21*+/-1,16** | **5,77*+/0,83** |
| | *100* | *79* | *55* | *51* |
| Ricinoléate de méthyle | 10,56 | 6,52 | 7,84 | 3,68 |
| | 12,00 | 8,36 | 7,40 | 4,52 |
| | 11,64 | 6,68 | 5,88 | 4,80 |
| | **11,40+/-0,75** | **7,19*+/-1,01** | **7,04*+/-1,03** | **4,33*+/-0,58** |
| | 100 | 63 | 62 | 38 |
| Les résultats sont exprimés en pmoles de 5α-DHT formées/puits de culture.<br>En gras : moyenne et écart type<br>En italique : pourcentage du groupe'DMSO 0,1% (v/v)' | | | | |

* Moyenne significativement différente du groupe 'DMSO 0,1% (v/v)'

**Exemple 6 : composition d'une crème pour l'hyperséborrhée**

| | % en poids |
|---|---|
| Esters butyliques d'huile d'avocat | 15,0 |
| Alcohol starylique | 3,0 |
| Steareth-21 | 3,0 |
| Steareth-2 | 2,0 |
| Eau | q.s.p. 100 |
| Carbopol | 0,4 |

(suite)

| | % en poids |
|---|---|
| Hydroxyde de sodium | 0,3 |
| Hydroxytoluène butylé (BHT) | 0,4 |
| Parfum | 0,1 |
| | **100 %** |

**Revendications**

1. Utilisation d'au moins un ester gras choisi parmi les oléates de méthyle, de butyle, d'oléyle ; le ricinoléate de méthyle ; les esters alkyliques d'huile d'avocat ou les mélanges de ces derniers pour la préparation d'une composition destinée au traitement de l'hypertrophie prostatique, de l'adénome prostatique, de l'acné, de l'hyperséborrhée, de l'alopécie, de l'hirsutisme.

2. Utilisation selon la revendication 1, **caractérisée en ce que** l'ester gras est obtenu par transestérification de triglycérides de l'huile d'avocat.

3. Utilisation selon la revendication 2, **caractérisée en ce que** l'ester gras est constitué des esters méthyliques de l'huile d'avocat.

4. Utilisation selon la revendication 2, **caractérisée en ce que** l'ester gras est constitué des esters butyliques de l'huile d'avocat.

5. Utilisation selon la revendication 1, **caractérisée en ce que** l'ester gras est l'oléate de butyle.

6. Utilisation selon la revendication 1, **caractérisée en ce que** l'ester gras est le ricinoléate de méthyle.

7. Utilisation selon l'une quelconque des revendications précédentes, **caractérisée en ce que** l'ester gras est utilisé selon une proportion comprise entre 0,001 et 100 % en poids, par rapport au poids total de la composition.

8. Utilisation selon l'une quelconque des revendications précédentes, **caractérisée en ce que** la composition préparée comprend un excipient pharmaceutiquement, dermatologiquement ou cosmétiquement acceptable.

9. Utilisation selon la revendication 8, **caractérisée en ce que** l'excipient est adapté pour une administration par voie topique externe ou par voie rectale.

10. Méthode de traitement cosmétique de la peau grasse, **caractérisée en ce qu'**on applique sur la peau une composition cosmétique contenant au moins un ester gras tel que défini à l'une quelconque des revendications 1 à 6.

11. Méthode de traitement cosmétique de la chute des cheveux, **caractérisée en ce qu'**on applique sur le cuir chevelu une composition cosmétique contenant au moins un ester gras tel que défini à l'une quelconque des revendications 1 à 6.

12. Méthode de traitement cosmétique de l'excès de pilosité, **caractérisée en ce qu'**on applique sur les zones de la peau présentant des excès de pilosité une composition cosmétique contenant au moins un ester gras tel que défini à l'une quelconque des revendications 1 à 6.

13. Méthode de traitement cosmétique selon l'une quelconque des revendications 10 à 12, **caractérisée en ce que** l'ester gras est présent dans la composition selon une proportion comprise entre 0,001 et 100% en poids, par rapport au poids total de la composition.

14. Méthode selon l'une quelconque des revendications 10 à 13, **caractérisée en ce que** la composition cosmétique contient en outre au moins un excipient cosmétiquement acceptable.

## Patentansprüche

1. Verwendung von wenigstens einem Fettester, ausgewählt aus den Methyl-, Butyl-, Oleyloleaten; Methylricinoleat; den Alkylestern von Avocadoöl oder den Mischungen von diesen Letzteren, für die Herstellung einer Zusammensetzung, die für die Behandlung von Prostatavergrößerung, Prostataadenom, Akne, Hyperseborrhoe, Alopezie, Hirsutismus bestimmt ist.

2. Verwendung nach Anspruch 1, **dadurch gekennzeichnet, dass** der Fettester durch Umesterung von Triglyceriden von Avocadoöl erhalten wird.

3. Verwendung nach Anspruch 2, **dadurch gekennzeichnet, dass** der Fettester aus Methylestern von Avocadoöl besteht.

4. Verwendung nach Anspruch 2, **dadurch gekennzeichnet, dass** der Fettester aus Butylestern von Avocadoöl besteht.

5. Verwendung nach Anspruch 1, **dadurch gekennzeichnet, dass** der Fettester Butyloleat ist.

6. Verwendung nach Anspruch 1, **dadurch gekennzeichnet, dass** der Fettester Methylricinoleat ist.

7. Verwendung nach einem der vorangegangenen Ansprüche, **dadurch gekennzeichnet, dass** der Fettester entsprechend einem Anteil zwischen 0,001 und 100 Gew.-% bezogen auf das Gesamtgewicht der Zusammensetzung eingesetzt wird.

8. Verwendung nach einem der vorangegangenen Ansprüche, **dadurch gekennzeichnet, dass** die hergestellte Zusammensetzung einen aus pharmazeutischer, dermatologischer oder kosmetischer Sicht annehmbaren Träger umfasst.

9. Verwendung nach Anspruch 8, **dadurch gekennzeichnet, dass** der Träger für eine Verabreichung auf topischem äußerlichem Wege oder auf rektalem Wege angepasst ist.

10. Verfahren zur kosmetischen Behandlung von fettiger Haut, **dadurch gekennzeichnet, dass** man auf die Haut eine kosmetische Zusammensetzung, die wenigstens einen Fettester, wie in einem der Ansprüche 1 bis 6 definiert, enthält, aufträgt.

11. Verfahren zur kosmetischen Behandlung von Haarausfall, **dadurch gekennzeichnet, dass** man auf die behaarte Haut eine kosmetische Zusammensetzung, die wenigstens einen Fettester, wie in einem der Ansprüche 1 bis 6 definiert, enthält, aufträgt.

12. Verfahren zur kosmetischen Behandlung von übermäßiger Behaarung, **dadurch gekennzeichnet, dass** man auf die Zonen der Haut, die übermäßige Behaarung aufweisen, eine kosmetische Zusammensetzung, die wenigstens einen Fettester, wie in einem der Ansprüche 1 bis 6 definiert, enthält, aufträgt.

13. Verfahren zur kosmetischen Behandlung nach einem der Ansprüche 10 bis 12, **dadurch gekennzeichnet, dass** der Fettester in der Zusammensetzung entsprechend einem Anteil zwischen 0,001 und 100 Gew.-% bezogen auf das Gesamtgewicht der Zusammensetzung vorhanden ist.

14. Verfahren nach einem der Ansprüche 10 bis 13, **dadurch gekennzeichnet, dass** die kosmetische Zusammensetzung außerdem wenigstens einen aus kosmetischer Sicht annehmbaren Träger enthält.

## Claims

1. Use of at least one fatty ester chosen from methyl oleate, butyl oleate, oleyl oleate; methyl ricinoleate; the alkyl esters of avocado oil or mixtures thereof, for the preparation of a composition designed for treating prostatic hypertrophy, prostatic adenoma, acne, hyperseborrhoea, alopecia and hirsutism.

2. Use according to Claim 1, **characterized in that** the fatty ester is obtained by transesterification of triglycerides

of avocado oil.

3. Use according to Claim 2, **characterized in that** the fatty ester consists of methyl esters of avocado oil.

4. Use according to Claim 2, **characterized in that** the fatty ester consists of butyl esters of avocado oil.

5. Use according to Claim 1, **characterized in that** the fatty ester is butyl oleate.

6. Use according to Claim 1, **characterized in that** the fatty ester is methyl ricinoleate.

7. Use according to any one of the preceding claims, **characterized in that** the fatty ester is used in a proportion of between 0.001 and 100% by weight, relative to the total weight of the composition.

8. Use according to any one of the preceding claims, **characterized in that** the composition prepared comprises a pharmaceutically, dermatologically or cosmetically acceptable excipient.

9. Use according to Claim 8, **characterized in that** the excipient is suitable for administration by the external topical route or by the rectal route.

10. Method for the cosmetic treatment of greasy skin, **characterized in that** a cosmetic composition containing at least one fatty ester as defined in any one of Claims 1 to 6 is applied to the skin.

11. Method for the cosmetic treatment of hair loss, **characterized in that** a cosmetic composition containing at least one fatty ester as defined in any one of Claims 1 to 6 is applied to the scalp.

12. Method for the cosmetic treatment of excess pilosity, **characterized in that** a cosmetic composition containing at least one fatty ester as defined in any one of Claims 1 to 6 is applied to the regions of the skin exhibiting excess pilosity.

13. Method of cosmetic treatment according to any one of Claims 10 to 12, **characterized in that** the fatty ester is present in the composition in a proportion of between 0.001 and 100% by weight, relative to the total weight of the composition.

14. Method according to any one of Claims 10 to 13, **characterized in that** the cosmetic composition contains, in addition, at least one cosmetically acceptable excipient.